# EUROPEAN PATENT SPECIFICATION

(11) **EP 1 307 603 B1**
(45) Date of publication and mention of the grant of the patent: **30.03.2005**
(21) Application number: 01963383.3
(22) Date of filing: 06.08.2001
(51) Int. Cl.: C22C 33/02, C23C 4/04, B65G 39/00

(54) **COMPOSITION FOR ELEMENTS HAVING A HIGH STRENGTH, IN PARTICULAR FOR HOT WEAR AND THERMAL FATIGUE, ROLLS COATED WITH SAID COMPOSITION**
ZUSAMMENSETZUNG FÜR HOCHFESTE ELEMENTE UND MITTELS DIESER ZUSAMMENSETZUNG BESCHICHTETE WALZE
COMPOSITION POUR ELEMENTS PRESENTANT UNE RESISTANCE ELEVEE NOTAMMENT A L'USURE THERMIQUE ET A LA FATIGUE THERMIQUE ET ROULEAUX REVETUS DE LADITE COMPOSITION

(30) Priority: 04.08.2000 IT RM20000443
(43) Date of publication of application: 07.05.2003
(73) Proprietor: CENTRO SVILUPPO MATERIALI S.p.A., 00129 Roma (IT)
(72) Inventor: GIMONDO, Pietro, Cen. Sviluppo Materiali S.p.A., I-00129 Roma (IT); CASADEI, Fabrizio, Cen. Sviluppo Materiali S.p.A., I-00129 Roma (IT); CAROSI, Andrea, Cen. Sviluppo Materiali S.p.A., I-00129 Roma (IT)
(74) Representative: Leone, Mario
(86) International application number: PCT/IT2001/000436
(87) International publication number: WO 2002/012579

(56) References cited:
- DE-A- 4 411 296
- US-A- 4 348 433
- PATENT ABSTRACTS OF JAPAN vol. 016, no. 383 (C-0974), 17 August 1992 (1992-08-17) -& JP 04 124255 A (KOBE STEEL LTD;OTHERS: 01), 24 April 1992 (1992-04-24)
- PATENT ABSTRACTS OF JAPAN vol. 1998, no. 10, 31 August 1998 (1998-08-31) -& JP 10 121221 A (DOI SEISAKUSHO:KK;UMAGOME MASAKATSU; FUKUDA METAL FOIL & POWDER CO), 12 May 1998 (1998-05-12)
- PATENT ABSTRACTS OF JAPAN vol. 012, no. 373 (C-534), 6 October 1988 (1988-10-06) -& JP 63 125652 A (KAWASAKI STEEL CORP), 28 May 1988 (1988-05-28)
- W.G. WOOD (COORDINATOR): "Metals Handbook. Ninth Edition. Vol. 5: Surface Cleaning Finishing and Coating" 1982 , AMERICAN SOCIETY FOR METALS , USA XP002184441 page 363
- PATENT ABSTRACTS OF JAPAN vol. 2000, no. 11, 3 January 2001 (2001-01-03) -& JP 2000 219911 A (DAI ICHI HIGH FREQUENCY CO LTD), 8 August 2000 (2000-08-08)

## Description

The present invention relates to a composition of a type suitable for the forming of elements for severe conditions of hot wear and thermal fatigue. In particular, this composition is suitable for forming a coating having a high strength, apt to form the outer layer of rolls subjected to severe working conditions of thermomechanical fatigue, in particular rolls for the handling of thick slabs ejected from an ingot mould in a steel mill.

The present invention further relates to rolls for the handling of thick slabs provided with such coating.

Though the invention generally relates to elements apt to withstand a wear, e.g., supporting elements and the like, reference will hereinafter be made to a type of element apt to incorporate the present invention and also to be by itself a subject thereof.

The rolls employed for the handling of the thick steel slabs, due to their peculiar working conditions, exhibit a gradual deterioration over time, until reaching a condition considered as unsuitable for their duty.

Therefore, in a continuous-cycle steel mill, in order to restore the surface of the rolls three to five yearly interventions are carried out.

These components are usually coated with welding technologies, deposing wear- and corrosion-resistant high Cr alloys.

With this technique, a steel strip is helically wound about the roll. The former is welded edge-to-edge.

It should be borne in mind that generally the progress rates of the thick slabs are in the order of 20 mm/sec., at temperatures of from 980°C to 1100°C. The rolls are generally cooled with atomised water jets.

In this context, the greater deterioration concentrates at the welded areas of the strip edges, these phenomena lead to an intensification of the deterioration itself.

Such phenomena are mainly stress corrosion due to the parallel presence of fatigue stress, from the rotation of the rolls contacting the thick slab, and of an aggressive environment, due to high temperatures, the acidity and the presence of water.

Another phenomenon causing wear and damage is the abovementioned cyclic thermal fatigue stress, from the contact of the roll with the hot thick slab, followed by the temporary cooling with water. Moreover, even the adhesive-abrasive wear, due to the roll-thick slab sliding, possibly in presence of-deposits, should not be underestimated.

In the field of the invention, powder composition for high-temperature aggregation processes and coating thereby obtained are known from JP 04 124255, JP 10 121221, JP 63125652, US 4,348,433 and DE44 11 296.

The technical problem underlying the present invention is that of providing a composition for elements having a high strength, e.g., the coating for rolls for the handling of thick slabs which allows for the elimination of the above described damage to more acceptable terms.

This problem is solved by a composition of powders for elements having a high strength, in particular to thermomechanical wear, comprising powders suitable for a high-temperature aggregation process, characterized in that it has percentages as resumed in the following Table:

| | **min (%/w)** | **max (%/w)** |
|---|---|---|
| Manganese (Mn) | 0.7 | 1.0 |
| Chromium (Cr) | 5.0 | 10.0 |
| Silicon (Si) | 0.4 | 0.6 |
| Carbon (C) | 0.35 | 0.40 |
| Nickel (Ni) | 0.5 | 0.7 |
| Molybdenum (Mo) | 1.6 | 2.0 |
| Cobalt (Co) | ∼ 0.2 | |
| Tungsten (W) | 0.4 | 0.5 |
| Iron (Fe) | q.s. to 100% | |

The present invention further relates to a coating of a type having a high strength to hot wear and to thermal fatigue, apt to form the outer layer of rolls subjected to severe working conditions to thermomechanical fatigue, in particular rolls (1) for the handling of thick slabs ejected from an ingot mold in a steel mill, deposed by high- temperature powder transfer, characterized in that the composition thereof has percentages as resumed in the following Table:

| | **min (%/w)** | **max (%/w)** |
|---|---|---|
| Manganese (Mn) | 0.7 | 1.0 |
| Chromium (Cr) | 5.0 | 10.0 |
| Silicon (Si) | 0.4 | 0.6 |
| Carbon (C) | 0.35 | 0.40 |
| Nickel (Ni) | 0.5 | 0.7 |
| Molybdenum (Mo) | 1.6 | 2.0 |
| Cobalt (Co) | ∼ 0.2 | |
| Tungsten (W) | 0.4 | 0.5 |
| Iron (Fe) | q.s. to 100% | |

A deposition method for a coating having a high strength comprises the following steps:
* preheating a receiving sublayer for said coating;
* providing a powdered material apt to form said coating, said material having a Chromium content ranging between 5% and 10% by weight;
* increasing the temperature of said powdered material and spraying it, in a high-temperature transfer flow, on said sublayer.

The main advantage of the composition and of the coating, according to the present invention, as well as of the method of deposition for the coating, is that they allow for a longer duration of the element having a high strength, with a minimisation over time of the induced surface defects.

Hereinafter, reference will be made to some preferred variant embodiments of the composition, of the coating and of the method of deposition for the coating, by way of example and without limitative purposes, from which further features and advantages thereof will be made clear.

Lastly, reference will be made to a test coating, applied on a suitable test piece.

Moreover, reference will be made to the attached drawings, wherein:
* Fig. 1 is a schematic representation in longitudinal section of a roll for the handling of thick slabs in a steel mill; and
* Fig. 2 is a schematic representation of a method of powder transfer applied to a roll of Fig. 1.

Various powder aggregation processes suitable for the composition according to the invention are known: sintering and melting, concerning elements entirely made of said composition, and powder transfer, essentially to form coatings.

The high-temperature powder transfer entails a process in which a powder flow is heated beyond the melting temperatures of the components of the powders themselves and sprayed on the bare surface of the roll to be coated. The powder spraying may follow, on said cylinder-shaped surface, a helical course.

Various powder transfer mechanisms are known. A first example of transfer is represented by a technology based on a flow having a high speed and temperature of a fuel-comburent association to which said powders are associated.

This technology is carried out in a torch inside which a fuel, e.g., kerosene, and gaseous oxygen are in a state of chemical combustion. The material with which the coating is to be carried out, is injected, in powder form, into the torch.

The presence of a convergent-divergent nozzle downstream of the combustion chamber implements the expansion of the hot gases up to a hypersonic speed (Mach " 1).

The partially fused particles are thus accelerated towards the surface of the roll to be coated, whereon they can form a thick coating remarkable for its high density. Moreover, no surface melting is induced on the sublayer receiving the coating, which thereby maintains its essential peculiarities.

Another example of high-temperature powder transfer is the plasma arc transfer. The powder is fused and sprayed onto the sublayer in association to an electric arc generated between a suitable torch and the component to be coated, a suitable potential difference being induced therebetween.

A plasma gas having a high temperature, protected in turn by a jet of inert gas provided for the purpose, e.g., Argon, is formed when passing through the electric arc.

The high temperatures reached in the electric arc and in the plasma allow the partial fusion of the sublayer and of the powders for the coating, respectively, leading to the deposition of a coating layer characterised by different dilution rates.

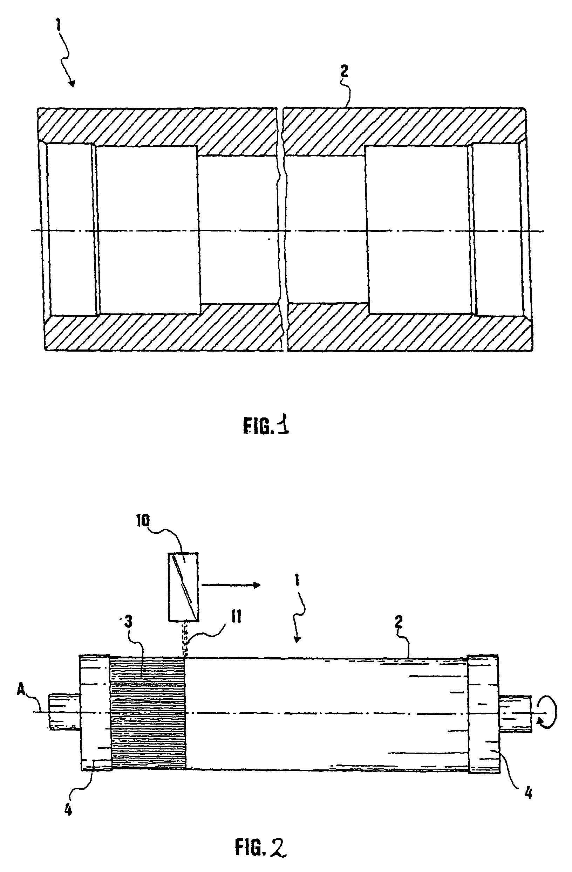

The thickness of the coating is generated by the sequence of consecutive passes of the torch according to a helical course onto the cylinder-shaped surface of the roll (Fig. 2).

Both techniques are applicable on a roll 1 of the kind employed for the handling of thick steel slabs manufactured in an ingot mould of a steel mill (Fig. 1). This roll 1 has an outer surface 2 constituting the sublayer onto which the coating 3 is applied.

In carrying out these techniques, a torch 10 generates a powder transfer flow 11 directly onto the cylinder-shaped surface 2 of roll 1.

The torch is shifted lengthwise with respect to roll 1, which is driven in rotation about its own axis A. The ends of roll 1, which are not subject to wearing off as unaffected by the transit of the thick slabs, are protected by bottoms 4.

In case of plasma arc transfer, bottoms 4 are made of copper and make the electrical connection necessary for obtaining the required potential difference. Bottoms 4 impede arcing, which instead goes off at the steel sublayer 2 of the roll 1.

The powders are preferably prepared as follows.

A molten steel having the desired composition is cast in a preheated cone-shaped tundish having a cylinder-shaped taphole bottom with a suitable diameter: in being outletted from the latter, the flow of molten steel is impinged upon by a high-pressure radial flow of inert gas, like Argon.

This gaseous flow atomises the melt, i.e., subdivides the melt into very fine droplets, which, upon cooling, turn into powder.

By adequately selecting the construction parameters of the abovesketched apparatus and the process parameters, like the casting temperature of the molten steel, the preheating temperature, the molten steel flow/gas flow volumetric ratio, the gas flow rate, etc., a powder within a predetermined granulometric range may be attained.

This range may be refined by sifting.

According to the present invention, the coating, besides Chromium in a percentage by weight comprised between 5% and 10%, is made of additional elements, always in form of powdered material.

According to a preferred variant embodiment of the coating, the powder material comprises percentages of Manganese, Silicon, Carbon, Nickel, Molybdenum, Cobalt, Tungsten, besides Chromium, plus Iron q.s. to 100%.

The preferred percentages are resumed in the following Table:

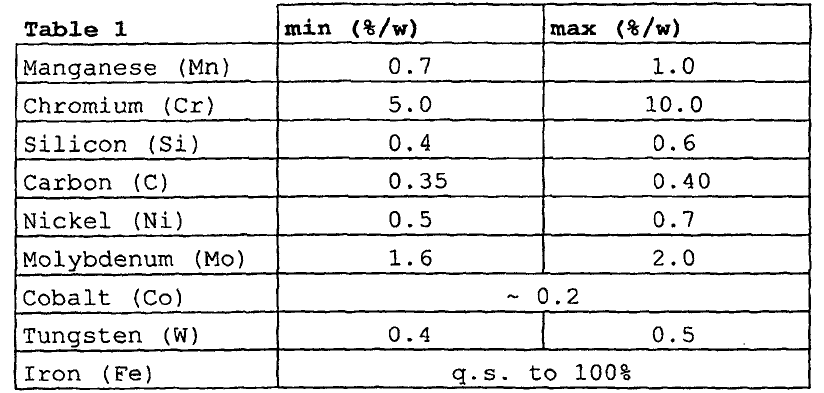

With reference to said ranges, a preferred composition for the material for the coating having a high strength is as follows: Mn: 0.8%; Cr: 5.8%; Si: 0.5%; C: 0.4%; Ni: 0.58%; Mo: 1.8%; Co: 0.2%; W: 0.4% and Fe q.s. to 100%, percentages by weight.

According to a preferred variant of the present invention, the transfer of the powders is carried out with the plasma arc technique.

In the execution of the method with this technique, the sublayer of the roll to be coated is preheated, preferably at a >250°C, and advantageously at a <450°C temperature.

Concerning the parameters to be adopted in the execution of said technique, the preferred values are reported in the following Table. For each parameter the measurement unit is indicated in brackets.

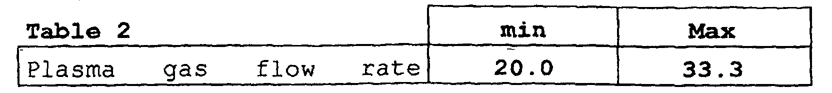

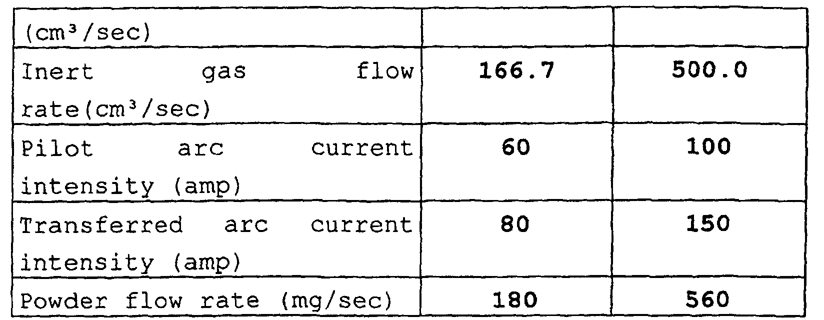

With reference to said ranges, the preferred approximate values of said parameters are as follows: - plasma gas flow rate: 26.67 cm³/sec; inert gas flow rate: 266.7 cm³/sec; pilot arc current intensity: 90 amp; transferred arc current intensity: 105 amp; and powder flow rate: 400 mg/sec.

As carrier gas, Argon, preferably mixed with Hydrogen which ensures the protection from oxidation combining to Oxygen and forming water, is employed. The quantity of Hydrogen with respect to the total carrier gas by volume is comprised between 4% and 15%. A preferred value for the former is about 10%.

The preferred granulometry for the powdered material ranges between 50 µm and 150 µm.

### Example

A hot wear test was conducted with a tool set in rotation at 200 rpm, for a time interval equivalent to about 50 days of continuous duty of a roll for the handling of thick slabs in ferritic steel, at the section adjacent to the taphole of the ingot mold. The same interval is equivalent to 30 days for thick slabs in austenitic steel.

The tool employed was made in ferritic steel provided with a coating deposed with a plasma arc powder transfer technique.

The composition of the coating is as follows: Mn: 0.8%; Cr: 5.8%; Si: 0.5%; C: 0.4%; Ni: 0.58%; Mo: 1.8%; Co: 0.2%; W: 0.4% and Fe q.s. to 100%, percentages by weight. The parameters for the coating technique were as follows: plasma gas flow rate: 26.67 cm³/sec; inert gas flow rate: 266.7 cm³/sec; pilot arc current intensity: 90 amp; transferred arc current intensity: 105 amp; and powder flow rate: 400 mg/sec.

As carrier gas, Argon mixed to 10% Hydrogen was employed. Moreover, a thermal fatigue test was conducted for 4•10³ cycles, in addition to those resulting from the wear test. For each cycle, the temperature was varied between 80°C and 800°C.

After these tests the coating was examined for surface defects, exhibiting optimum integrity (Fig. 3).

## Claims

1. A composition of powders for elements having a high strength, in particular to thermomechanical wear, comprising powders suitable for a high-temperature aggregation process, having percentages as resumed in the following Table:

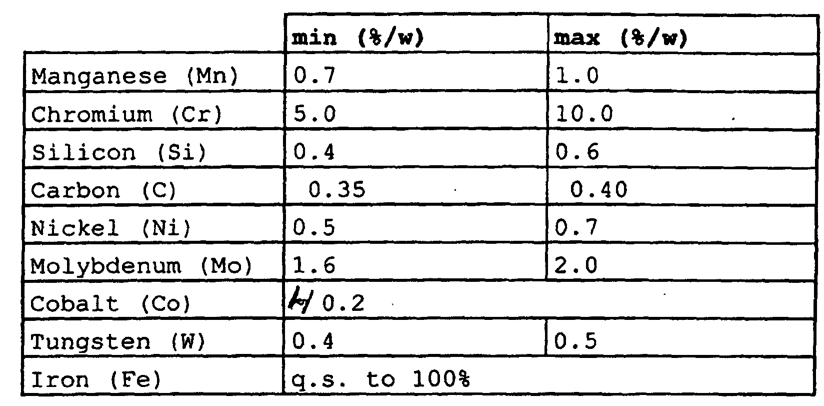

2. The composition according to claim 1, comprising: Mn: 0.8%; Cr: 5.8%; Si: 0.5%; C: 0.4%; Ni: 0.58%; Mo: 1.8%; Co: 0.2%; W: 0.4% and Fe q.s. to 100%, percentages by weight.

3. A coating (3) of a type having a high strength to hot wear and to thermal fatigue, apt to form the outer layer of rolls subjected to severe working conditions to thermomechanical fatigue, in particular rolls (1) for the handling of thick slabs ejected from an ingot mold in a steel mill, deposed by high-temperature powder transfer, wherein the composition thereof has percentages as resumed in the following Table:
| | **min (%/w)** | **max (%/w)** |
|---|---|---|
| Manganese (Mn) | 0.7 | 1.0 |
| Chromium (Cr) | 5.0 | 10.0 |
| Silicon (Si) | 0.4 | 0.6 |
| Carbon (C) | 0.35 | 0.40 |
| Nickel (Ni) | 0.5 | 0.7 |
| Molybdenum (Mo) | 1.6 | 2.0 |
| Cobalt (Co) | 0.2 | |
| Tungsten (W) | 0.4 | 0.5 |
| Iron (Fe) | q.s. to 100% | |

4. The coating (3) according to claim 3, wherein said composition is as follows: Mn: 0.8%; Cr: 5.8%; Si: 0.5%; C: 0.4%; Ni: 0.58%; Mo: 1.8%; Co: 0.2%; W: 0.4% and Fe q.s. to 100%, percentages by weight.

5. The coating (3) according to claims 3 or 4, wherein said high-temperature powder transfer is carried out with a plasma arc.

6. A roll (1) for the handling of thick slabs, comprising a coating (3) as set forth in any of the claims 3 to 5.

## Patentansprüche

1. Zusammensetzung von Pulvern für Elemente mit einer hohen Festigkeit, insbesondere gegenüber thermomechanischem Verschleiß, umfassend Pulver, die sich für einen Hochtemperatur-Aggregationsprozess eignen, mit Prozentgehalten, die in der folgenden Tabelle zusammengefasst sind:

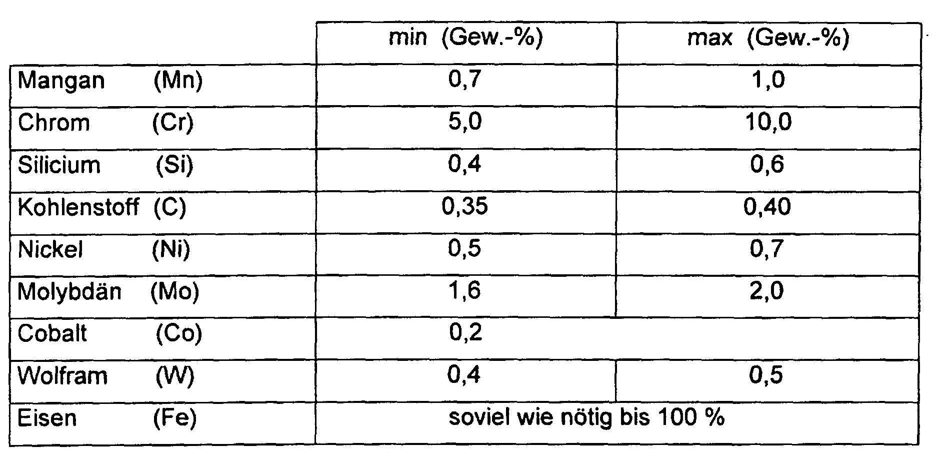

2. Zusammensetzung nach Anspruch 1, umfassend:
Mn: 0,8 %; Cr: 5,8 %; Si: 0,5 %; C: 0,4 %; Ni: 0,58 %; Mo: 1,8 %; Co: 0,2 %;
W: 0,4 % und Fe soviel wie nötig bis 100 %, wobei sich die Prozentgehalte auf das Gewicht beziehen.

3. Überzug (3) eines Typs mit einer hohen Festigkeit gegenüber Warmverschleiß und gegenüber thermischer Ermüdung, geeignet zum Bilden der Außenschicht von Walzen, die harten Arbeitsbedingungen mit thermomechanischer Ermüdung ausgesetzt sind, insbesondere Walzen (1) für die Handhabung von dicken Brammen, die aus einer Blockkokille in einem Stahlwerk ausgestoßen werden, abgeschieden durch Hochtemperatur-Pulvertransfer, wobei die Zusammensetzung davon Prozentgehalte aufweist, die in der folgenden Tabelle zusammengefasst sind:

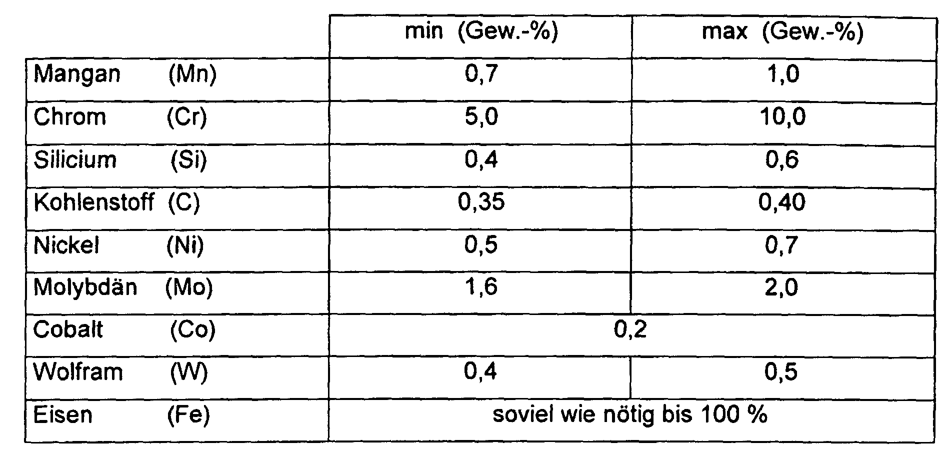

4. Überzug (3) nach Anspruch 3, wobei die Zusammensetzung wie folgt ist:
Mn: 0,8 %; Cr: 5,8 %; Si: 0,5 %; C: 0,4 %; Ni: 0,58 %; Mo: 1,8 %; Co: 0,2 %;
W: 0,4 % und Fe soviel wie nötig bis 100 %, wobei sich die Prozentgehalte auf das Gewicht beziehen.

5. Überzug (3) nach den Ansprüchen 3 oder 4, wobei der Hochtemperatur-Pulvertransfer mit einem Plasmabogen erfolgt.

6. Walze (1) für die Handhabung von dicken Brammen, umfassend einen Überzug (3) wie er in einem der Ansprüche 3 bis 5 angegeben ist.

## Revendications

1. Composition de poudres pour des éléments présentant une grande résistance, en particulier à la fatigue thermomécanique, comprenant des poudres convenant à un procédé de compaction à haute température, ayant des pourcentages tels que résumés dans le tableau suivant :

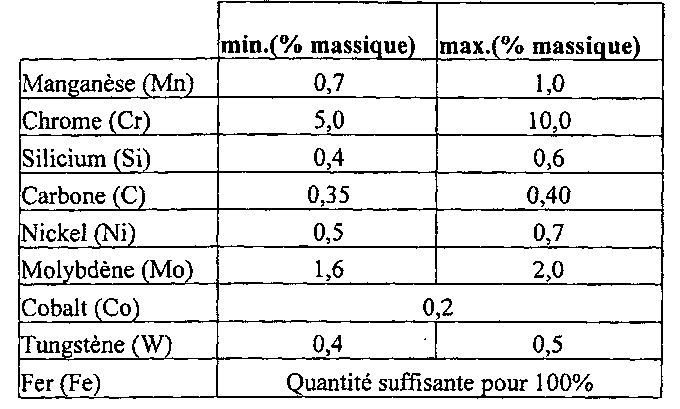

2. Composition selon la revendication 1, comprenant en pourcentages massiques : Mn : 0,8% ; Cr : 5,8% ; Si : 0,5%; C : 0,4% ; Ni : 0,58%; Mo : 1,8%; Co : 0,2% ; W : 0,4% et Fe en quantité suffisante pour 100%,.

3. Revêtement (3) d'un type présentant une grande résistance à l'usure à chaud et à la fatigue thermique, apte à former la couche extérieure de rouleaux soumis à de sévères conditions de fonctionnement en fatigue thermomécanique, en particulier des rouleaux (1) pour la manutention de brames épaisses éjectées d'une lingotière vers un broyeur d'acier, déposé par transfert de poudre à haute température, dont la composition a des pourcentages tels que résumés dans le tableau suivant :

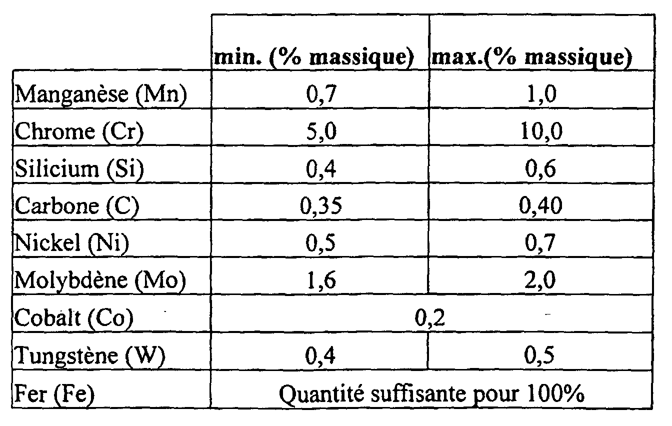

4. Revêtement (3) selon la revendication 3, pour lequel ladite composition en pourcentages massiques est comme suit : Mn : 0,8% ; Cr : 5,8% ; Si : 0,5% ; C : 0,4%; Ni: 0,58%; Mo: 1,8%; Co : 0,2% ; W : 0,4% et Fe en quantité suffisante pour 100%,.

5. Revêtement (3) selon l'une des revendications 3 ou 4, pour lequel ledit transfert de poudre à haute température est effectué avec un arc à plasma.

6. Rouleau (1) pour la manutention de brames épaisses, comprenant un revêtement (3) tel que revendiqué dans l'une quelconque des revendications 3 à 5.
